# FASCICULE DE BREVET EUROPEEN

(11) **EP 2 643 448 B1**
(45) Date de publication et mention de la délivrance du brevet: **11.03.2015**
(21) Numéro de dépôt: 11788441.1
(22) Date de dépôt: 25.11.2011
(51) Int. Cl.: C12M 1/00, F24J 2/06, F24J 2/07

(54) **PHOTOBIORÉACTEUR SOLAIRE À DILUTION CONTRÔLÉE DU FLUX EN VOLUME**
SOLAR-PHOTOBIOREAKTOR MIT KONTROLLIERTER VOLUMENSTROMVERDÜNNUNG
SOLAR PHOTOBIOREACTOR WITH CONTROLLED VOLUME FLOW DILUTION

(30) Priorité: 25.11.2010 FR 1059761
(43) Date de publication de la demande: 02.10.2013
(73) Titulaire: Centre National de la Recherche Scientifique (CNRS), 75016 Paris (FR); Ecole Nationale Superieure de Chimie de Clermont-Ferrand, 63170 Aubiere (FR); Université Blaise Pascal Clermont II, 63000 Clermont Ferrand (FR)
(72) Inventeur: CORNET, Jean-François, F-63830 Durtol (FR); GOETZ, Vincent, F-66450 Pollestres (FR); PLANTARD, Gaël, F-11100 Narbonne (FR); GARCIA, Roger, F-66330 Cabestany (FR); LAFON, Pascal, F-63970 Aydat (FR); JOYARD, Frédéric, F-63450 Saint Saturnin (FR)
(74) Mandataire: Regimbeau
(86) Numéro de dépôt international: PCT/EP2011/071002
(87) Numéro de publication internationale: WO 2012/069622

(56) Documents cités:
- WO-A2-2007/134141
- WO-A2-2010/011299
- US-A1- 2010 144 019
- CORNET: "Calculation of optimal design and ideal productivities of volumetrically lightened photobioreactors using the construal approach", CHEMICAL ENGINEERING SCIENCE, vol. 65, no. 2, 16 janvier 2010 (2010-01-16), pages 985-998, XP002647138,

## Description

### Domaine de l'invention

L'invention concerne le domaine général des photo-réacteurs. Plus particulièrement, invention concerne le domaine des photo-bioréacteurs et des modules de captation et de répartition de lumière solaire pour photo-bioréacteurs utilisés pour la production de microorganismes photosynthétiques.

### Arrière plan technologique

Aujourd'hui deux techniques sont employées pour la production de microorganismes photosynthétiques : les techniques à ciel ouvert (par exemple : bassins ouverts) et les techniques en réacteur clos.

Les techniques en réacteur clos ont L'avantage de permettre :
- le maintien de la culture de microorganismes photosynthétiques, stérile évitant ainsi au moins les contaminations ;
- le contrôle strict des apports d'eau et de nutriments ;
- le contrôle du transfert de dioxyde de carbone et conjointement du pH, augmentant la productivité surfacique ;
- la culture à des concentrations en biomasse élevées augmentant également la productivité, mais aussi optimisant la compacité, ceci ayant pour résultat la diminution des coûts énergétiques associés à la circulation des fluides et au contrôle thermique du réacteur.

Les techniques en réacteur clos sont généralement réalisées par des photo-bioréacteurs à captation directe de la lumière du soleil à travers une paroi transparente.

Cependant, cette situation où la densité de flux solaire directe incidente est subie conduit à fonctionner avec une efficacité thermodynamique de conversion de la photosynthèse très faible. Ces techniques nécessitent donc une disponibilité en surface importante.

Il est également connu qu'utiliser des photo-bioréacteurs pour lesquels la lumière du soleil est d'abord captée puis amenée à l'intérieur des photo-bioréacteurs pour y être distribué à une faible densité de flux peut augmenter significativement l'efficacité thermodynamique de la photosynthèse, comme dans le document WO 2007/134141. Cela augmente d'autant la productivité en surface des photo-bioréacteurs. La captation peut être réalisée de diverse manière et permet de former un faisceau concentrant la lumière du soleil. Ce faisceau est ensuite renvoyé vers un guide de lumière pour amener le flux lumineux dans la chambre de réaction du photo-bioréacteur et y être distribué.

Cependant, dans un tel photo-bioréacteur, le guide ou diffuseur ne permet pas de diffuser de manière homogène la lumière à l'intérieur du photo-bioréacteur, diminuant ainsi l'efficacité du photo-bioréacteur. Également, le guide ou diffuseur peut subir des détériorations dues à une montée en température en leur sein trop importante les faisant parfois fondre. Enfin, la présence de nombreuses structures diffusant la lumière au sein de la chambre de réaction peut être à l'origine de problèmes d'adhésion cellulaire ou bien d'un mauvais mélange entraînant une perte d'efficacité.

### Présentation

Un des objectifs de l'invention est de pallier au moins un inconvénient de l'art antérieur.

Notamment, l'invention a pour objectif de permettre une meilleure efficacité de la captation directe du soleil en proposant un module de captation et de répartition de la lumière du soleil pour un photo-réacteur comprenant une chambre de réaction, le module comportant :
- un concentrateur disposé en dehors de la chambre à réaction pour concentrer la lumière solaire en un faisceau concentré ;
- un diffuseur à disposer dans la chambre de réaction pour introduire le faisceau concentré à l'intérieur de la chambre de réaction et diffuser le faisceau concentré à l'intérieur de la chambre de réaction ;
caractérisé en ce que le module comporte en outre un homogénéisateur disposé entre le concentrateur et le diffuseur, en dehors de la chambre de réaction, pour homogénéiser le faisceau concentré avant que le faisceau concentré entre dans le diffuseur.

Ce module a pour avantage de permettre l'homogénéisation du faisceau concentré par le concentrateur sur le diffuseur. En effet, il a été découvert que l'inhomogénéité de la diffusion de la lumière à l'intérieur du photo-réacteur était en partie due à l'inhomogénéité de l'éclairement du diffuseur par le faisceau concentré. En effet, un faisceau concentré a un profil de densité de flux de forme gaussienne. Ainsi le centre du diffuseur reçoit trop de lumière alors que sa périphérie n'en reçoit pas suffisamment. Le centre du diffuseur peut alors chauffer de manière excessive et fondre.

D'autres caractéristiques optionnelles et non limitatives sont :
- le module comprend en outre un guide de lumière pour guider la lumière du concentrateur au diffuseur, le guide de lumière étant disposé entre l'homogénéisateur et le diffuseur en dehors de la chambre de réaction.
- le module comprend en outre un filtre proche infrarouge pour filtrer la lumière du soleil de sorte que la lumière du soleil est exempte de rayonnement dans les proches infrarouges ;
- le concentrateur est un concentrateur à lentille de Fresnel comprenant une lentille de Fresnel ayant un foyer ;
- le module peut comprendre un capteur à miroir composé à foyer quasi-ponctuel disposé entre le concentrateur à lentille de Fresnel et l'homogénéisateur et devant le foyer de la lentille de Fresnel pour rediriger le faisceau concentré ;
- le concentrateur est un concentrateur de type Cassegrain ;
- le guide de lumière et le diffuseur forment un seul et même élément composé de fibres optiques ;
chaque fibre optique possédant une partie diffusante, les parties diffusantes des fibres optiques formant le diffuseur ; et
chaque fibre optique possédant une partie de guidage, les parties de guidage des fibres optiques rassemblées en un toron formant le guide de lumière.

L'invention propose également un panneau de captation et de répartition de la lumière du soleil comprenant au moins deux modules tels que décrits ci-dessus comprenant un concentrateur à lentille de Fresnel.

L'invention propose encore un photo-réacteur, notamment photo-bioréacteur, comprenant une chambre de réaction, caractérisé en ce qu'il comprend en outre un module ou un panneau tels que décrits ci-dessus.

Un avantage avec un tel photo-réacteur est lié à l'utilisation du module de guidage de lumière. Les avantages du module sont donc apportés à un tel photo-réacteur. De plus, grâce à la possibilité de regrouper les modules en panneau, le photo-réacteur peut avoir des dimensions diverses et le nombre de modules utilisés devant simplement être adapté.

D'autres caractéristiques optionnelles et non limitatives de ce photo-réacteur sont :
- les parties diffusantes des fibres optiques du module ou du panneau étant disposées tendues et parallèles entre elles à l'intérieur de la chambre de réaction suivant un maillage triangulaire ;
- le photo-réacteur comprend en outre au moins trois grilles disposées parallèlement l'une au-dessus de l'autre, chacune étant formée d'un cadre et de fils tendus à l'intérieur du cadre parallèlement les uns aux autres suivant une direction, la direction des fils d'une grille formant avec la direction des fils d'une grille au-dessus un angle de ±60° afin de maintenir les parties diffusantes des fibres optiques selon un maillage triangulaire ; et
- le photo-réacteur comprend en outre un gazosiphon à boucle de recirculation externe et un retour central pour mélanger efficacement le contenu de la chambre de réaction.

### Présentation des dessins

D'autres caractéristiques, objectifs et avantages apparaîtront à la lecture de la description détaillée qui suit, en référence aux dessins donnés à titre illustratif et non limitatif, parmi lesquels :
- la figure 1 est une vue schématique d'un exemple d'un photo-bioréacteur de l'invention ;
- la figure 2 est une vue de trois-quarts d'un premier mode de réalisation d'un module de guidage de lumière avec un concentrateur à lentille de Fresnel pour un photo-bioréacteur ;
- la figure 3 est une vue en coupe du module de la figure 1 ;
- la figure 4 est une vue de trois-quarts d'un panneau composé de plusieurs modules de la figure 1 ;
- la figure 5 est une vue en trois-quarts d'un ensemble photo-bioréacteur et panneau du type du panneau de la figure 4 ;
- la figure 6 est une vue en trois-quarts d'un ensemble photo-bioréacteur et module de guidage de lumière selon un deuxième mode de réalisation avec un concentrateur Cassegrain ;
- la figure 7 est une vue partielle en trois-quarts d'un mode de réalisation d'un homogénéisateur utilisé dans les modules représentés sur les figures 1 à 6 ;
- la figure 8 est une vue approchée d'un regroupement de fibres optiques en torons tel qu'utilisé avec les modules des figures 1 à 6 ;
- la figure 9 est une vue en trois-quarts d'un grillage à placer à l'intérieur de la chambre de réaction d'un photo-bioréacteur pour une répartition parallèle des fibres optiques en maille triangulaire ;
- la figure 10 est une vue de l'intérieur de la chambre de réaction dans laquelle cinq grilles de la figure 9 sont utilisées pour répartir les fibres optiques ;
- la figure 11 est une vue en trois-quarts et partiellement découpée d'un capteur utilisé dans les modules des figures 1 à 5 ;
- la figure 12 est une vue en trois-quarts d'un exemple de suiveur du soleil utilisé avec les modules des figures 1 à 6 ;
- la figure 13 est une vue en coupe transversale d'une chambre de réaction entre deux grilles et montrant la répartition de fibres optiques selon une maille triangulaire à l'intérieur de la chambre de réaction.

### Description détaillée

### Module de captation et de répartition de la lumière du soleil

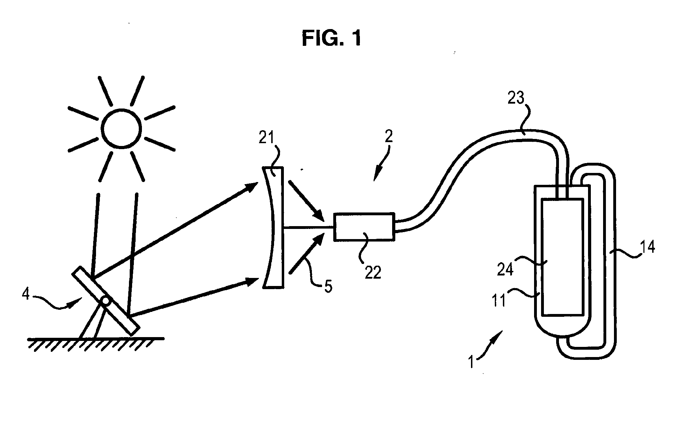

En référence aux figures 1 à 6 et 10 est décrit ci-après un module **2** de captation et de répartition de lumière solaire pour photo-bioréacteur.

Le module **2** a pour rôle de capter un flux lumineux provenant de la lumière du soleil et de le transporter à l'intérieur d'un photo-bioréacteur **1** comprenant une chambre **11** de réaction et de répartir le flux lumineux capté de manière la plus homogène possible dans la chambre **11** de réaction.

La chambre **11** de réaction est un espace intérieur du photo-bioréacteur **1** dans laquelle sont placés les microorganismes en culture dans une solution. Les microorganismes y reçoivent le flux lumineux et les nutriments nécessaires à leur croissance et développement.

Le module **2** comprend un concentrateur **21** pour concentrer le flux lumineux provenant de la lumière du soleil en un faisceau concentré **5.** Le concentrateur **21** peut avoir plusieurs modes de réalisation qui seront décrits plus loin.

Le module **2** comprend également un diffuseur **24** à placer à l'intérieur de la chambre **11** de réaction pour y diffuser le flux lumineux du faisceau concentré **5.**

Le module **2** comprend encore un guide de lumière **23,** à placer à l'extérieur de la chambre **11** de réaction, pour conduire le flux lumineux du faisceau concentré **5** du concentrateur **21** au diffuseur **24.**

Le flux lumineux du faisceau concentré **5** peut être très élevé dans une zone proche du centre du faisceau concentré **5.** Dans la plupart des cas, le flux lumineux du faisceau concentré **5** possède un profil gaussien.

Lorsque le faisceau concentré **5** entre dans le guide de lumière **23,** cette zone à flux lumineux élevé peut endommager voire détruire le guide de lumière **23** et également le diffuseur **24.** Dans le cas de fibres optiques **25** jouant le rôle de guide de lumière **23** et de diffuseur **24,** comme décrit par la suite, la zone à flux lumineux élevé peut faire fondre les fibres optiques **25.**

Afin d'éviter l'endommagement du guide de lumière **23** et/ou du diffuseur **24,** le module **2** comprend encore un homogénéisateur **22** pour homogénéiser le flux lumineux du faisceau concentré **5** sur le guide de lumière **23.**

L'homogénéisateur **22** permet d'uniformiser le flux lumineux du faisceau concentré **5** en aplanissant son profil. De ce fait, le risque de surchauffe dans la zone à flux lumineux élevé du faisceau concentré **5** est également évité.

L'homogénéisateur **22** peut être réalisé sous la forme d'un barreau optique. L'uniformisation du profil de la densité de flux de la lumière du faisceau concentré **5** est obtenue par de multiples réflexions quasi totales que font les rayons du faisceau concentré **5** à l'intérieur du barreau optique **22** avant d'en sortir. En effet, les rayons du faisceau concentré **5** entrent dans le barreau optique **22** avec des angles différents et suivent des chemins optiques différents à l'intérieur du barreau optique **23.**

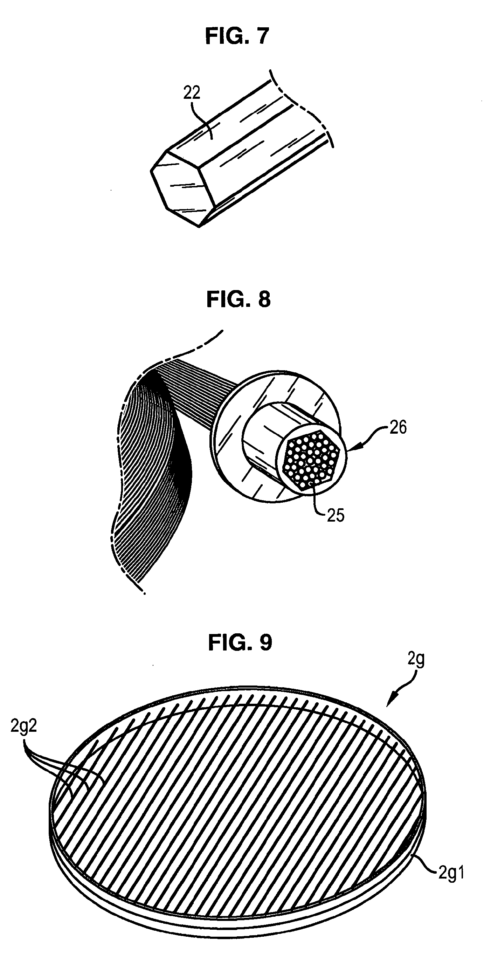

La longueur et la forme du barreau optique **22** peuvent varier. Par exemple, le barreau optique **22** est de forme parallélépipédique ou pyramidale. Le barreau optique **22** peut encore être un cylindre droit à base hexagonale (voir figure **7**).

Le barreau optique **22** peut être réalisé en verre de qualité optique, par exemple de type BK7.

On décrit ci-après un mode particulier de réalisation du guide de lumière **23** et du diffuseur **24.**

Dans ce mode particulier de réalisation, le guide de lumière **23** et le diffuseur **24** sont formés d'un ensemble de fibres **25** optiques, par exemple en polyméthacrylate de méthyle (PMMA) permettant de supporter des températures de chauffe du diffuseur allant au maximum jusqu'à 80°C environ.

Chaque fibre optique **25** possède une partie de guidage **25-23** et une partie diffusante **25-24.** L'ensemble des parties de guidage **25-23** des fibres optiques **25** forme alors le guide de lumière **23** et l'ensemble des parties diffusantes **25-24** des fibres optiques **25** forme le diffuseur **24.**

Les fibres optiques **25** entrent dans la chambre 11 de réaction par l'intermédiaire d'un couvercle **13** assurant l'étanchéité sur chaque fibre optique **25.**

Le faisceau concentré **5** entre par l'extrémité libre de la partie de guidage **25-23** des fibres optiques **25** et ressort de manière diffuse le long de la partie diffusante **25-24.**

La partie diffusante **25-24** des fibres optiques **25** est traitée au moins partiellement et avantageusement entièrement pour lui conférer des propriétés diffusives.

La partie diffusante **25-24** des fibres optiques **25** peut être traitée en outre par entourage de gaine individuelle en plastique qui sert à permettre la diffusion du flux lumineux dans la solution aqueuse. Ainsi, on maintient une couche d'air entre la gaine individuelle en plastique et les rugosités de la partie diffusante **25-24** des fibres optiques **25** occasionnées par le traitement laser, ce qui permet une diffusion du flux lumineux dans les liquides, lorsque le matériau utilisé pour les fibres optiques **25** ne le permet pas. La gaine en matière plastique est traitée à ses extrémités pour être étanche. Au niveau du couvercle **13** du photo-bioréacteur 1, les gaines individuelles sont surmoulées par une résine afin de les solidariser et d'étanchéifier le couvercle **13.**

La partie diffusante **25-24** des fibres optiques **25** a alors la propriété de diffuser la lumière radialement sur toute sa longueur jouant le rôle de diffuseur **24.**

L'avantage d'utiliser des fibres optiques **25** tient en ce qu'elles peuvent être réparties de manière optimale à l'intérieur de la chambre 11 de réaction du photo-bioréacteur **1,** comme décrit par la suite. En outre, l'utilisation de gaines individuelles en plastique plus rigide que le matériau des fibres optiques **25** permet un positionnement des partie diffusantes **25-24** plus aisé à l'intérieur de la chambre **11** de réaction lorsque les fibres optiques **25** sont trop souples.

Les parties de guidage **25-23** des fibres optiques **25** peuvent être assemblées en torons **26** (voir la figure 8), les parties diffusantes **25-24** des fibres optiques **25** à l'intérieur de la chambre 11 de réaction étant individualisées.

Des grilles **2g** peuvent être prévues pour être placées de manière régulière à l'intérieur de la chambre **11** de réaction.

Par exemple, lorsque le photo-bioréacteur **1** est de forme générale cylindrique à section droite en forme de disque et dont la longueur est positionnée suivant la verticale, chaque grille **2g** est avantageusement formée d'un cadre **2g1** circulaire et de fils **2g2** parallèles tendus à l'intérieur du cadre **2g1** et dont les extrémités sont fixées au cadre **2g1** circulaire. Le cadre **2g1** a plus ou moins les dimensions de la section droite de la chambre **11** de réaction également cylindrique.

Le cadre **2g1,** qui ci-dessus est circulaire, peut avoir une autre forme épousant les bords de la chambre **11** de réaction du photo-bioréacteur considérés.

Ces grilles **2g** permettent d'obtenir un maillage triangulaire des parties diffusantes **25-24** des fibres optiques **25,** comme il sera expliqué par la suite.

Le module **2** peut comprendre en outre un filtre **27** proche infrarouge pour filtrer les longueurs d'onde infrarouge et/ou proche infrarouge de la lumière solaire ou du faisceau concentrée **5.**

Le filtre **27** permet d'éviter l'entrée de rayonnement du domaine du proche infrarouge (700 nm et au-delà) inutile pour la photosynthèse des microorganismes (seule la lumière visible - entre 400 et 700 nm - est utile) et qui peut entraîner une augmentation excessive de la température au sein de la chambre **11** de réaction du photo-bioréacteur 1. L'augmentation de la température dans la chambre **11** de réaction au-delà d'un certain seuil peut tuer les microorganismes. L'augmentation de la température peut également avoir lieu dans le diffuseur **24** et donc le détériorer ; ainsi l'utilisation d'un filtre **27** diminue d'autant le risque de détérioration du diffuseur **24.**

### Concentrateur à lentille de Fresnel

### ▪ Module

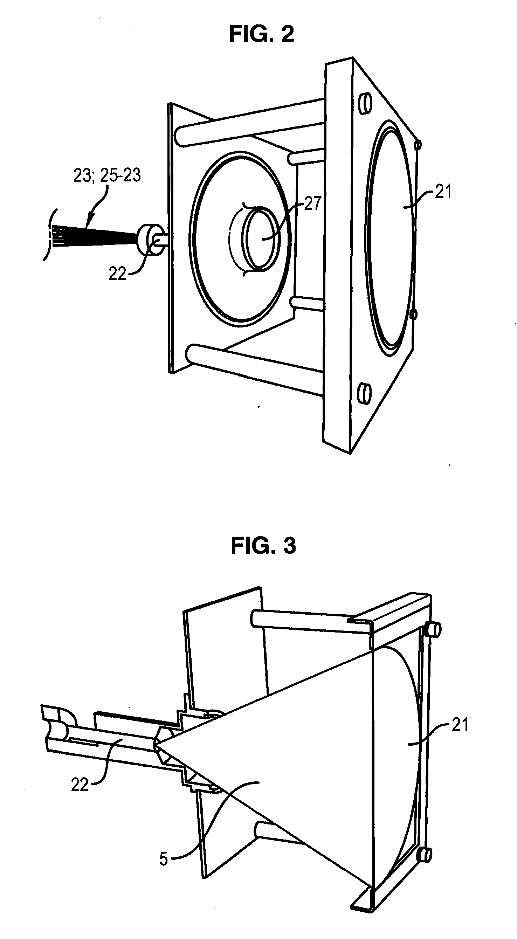

Dans un premier mode de réalisation particulière du module **2** décrit ci-dessus et illustré sur les figures 2 et 3, le concentrateur **21** peut être constitué d'une lentille convergente. La lentille convergente peut être une lentille de Fresnel.

L'utilisation d'une lentille de Fresnel permet d'obtenir un concentrateur **21** léger et facile à implanter. L'utilisateur d'une lentille de Fresnel permet également de réduire les coûts de fabrication du module **2.** Une lentille de Fresnel permet encore des taux de concentration de plusieurs centaines. La taille d'une lentille de Fresnel peut être comprise entre plusieurs millimètres à plusieurs dizaines de centimètres.

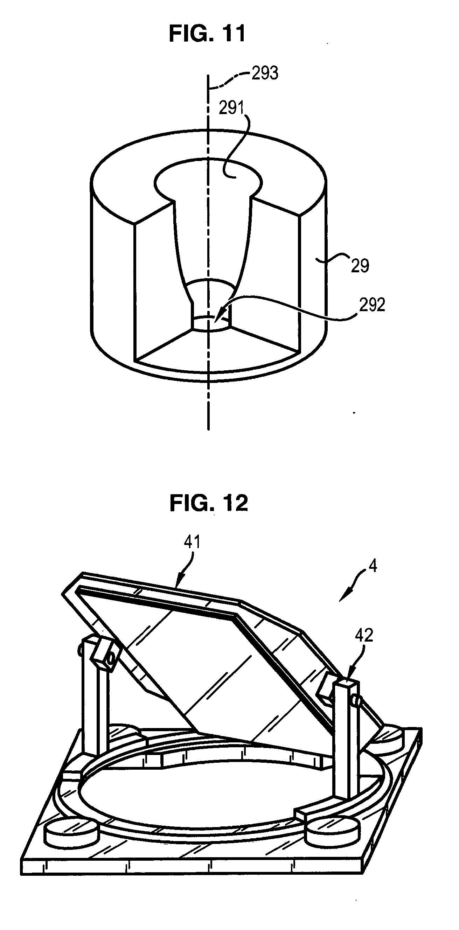

Un suiveur de soleil **4** (voir figure 12) est également prévu pour diriger les rayons du soleil vers le concentrateur **21** à lentille de Fresnel. Le suiveur de soleil **4** est positionné en face du concentrateur **21** à lentille de Fresnel. Le suiveur de soleil **4** comprend un miroir **41** dont l'inclinaison est orientable grâce à un régulateur d'inclinaison **42** et dont le plan de la surface intersecte horizontalement le plan moyen de la lentille de Fresnel du concentrateur **21.**

Un capteur **29** à miroir composé à foyer quasi-ponctuel (ou *« compound parabolic concentrator* » en anglais) illustré sur la figure 11 peut être prévu pour rediriger les rayons provenant de la lentille de Fresnel. Le capteur **29** est placé juste devant le foyer de la lentille de Fresnel, entre la lentille de Fresnel et l'homogénéisateur **22.**

Le capteur **29** à miroir composé à foyer quasi-ponctuel présente une symétrie de révolution et comporte une ouverture d'entrée **291** et une ouverture de sortie **292** centrées sur son axe de révolution **293.** La forme géométrique du capteur **29** est construite sur la base d'un arc de parabole et assure la redirection de tout rayon entrant dans l'ouverture d'entrée **291** vers l'ouverture de sortie **292** et donc vers l'homogénéisateur **22.** L'utilisation d'un tel capteur **29** permet une surconcentration d'un facteur allant de deux à quatre. Ce capteur **29** permet également de compenser un suivi du soleil de faible précision. L'ouverture d'entrée **291** du capteur **29** est suffisamment grande pour s'assurer que tout le faisceau concentré **5** par la lentille de Fresnel y entre.

Le filtre **27** peut être un film absorbant les longueurs d'onde infrarouge/proche infrarouge. Le filtre **27** peut encore être un miroir chaud qui présente la propriété de réfléchir les longueurs d'onde proche infrarouge et d'être transparent à la lumière visible utile pour la photosynthèse.

Le filtre **27** peut être positionné entre le concentrateur **21** et le capteur **29.** Le filtre **27** peut encore être positionné devant le concentrateur **21** qui ne reçoit alors qu'une lumière filtrée.

Dans le cas du concentrateur **21** à lentille de Fresnel, plusieurs modules **2** peuvent être rassemblés en parallèle et utilisés pour un même photo-bioréacteur **1.**

### ▪ Panneau de captation et de répartition de la lumière du soleil

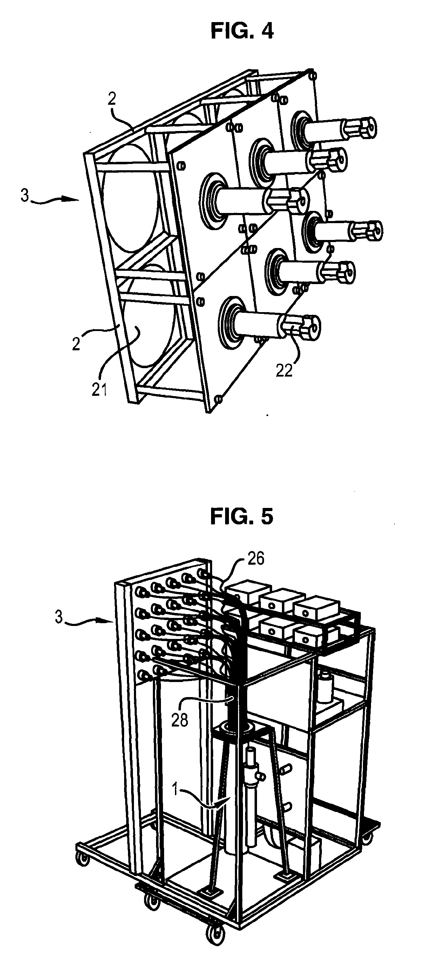

Plusieurs modules **2** tels que décrits ci-dessus peuvent être assemblés pour former un panneau **3** de captation et de répartition de la lumière du soleil, comme représenté sur les figures **4** et 5.

Dans un tel panneau **3,** les modules **2** sont montés en parallèle.

Lorsque le guide de lumière **23** et le diffuseur **24** sont formés par des fibres optiques **25** comme décrit ci-dessus, les parties de guidage **25-23** des fibres optiques **25** d'un module sont regroupées en torons **26.**

Les torons **26** de l'ensemble des modules **2** sont ensuite regroupés au niveau du couvercle **13** du photo-bioréacteur 1, les parties diffusantes **25-24** des fibres optiques **25** de tous les modules **2** étant laissées individualisées.

Par exemple, vingt-cinq (25) modules **2** sont utilisés pour un photo-bioréacteur **1.** Neuf-cent-soixante-dix-sept (977) fibres optiques **25** sont réparties à l'intérieur de la chambre **11** de réaction du photo-bioréacteur **1.** Les 977 fibres optiques **25** sont réparties en vingt-cinq (25) torons **26** de trente-neuf ou quarante (39 ou 40) fibres optiques **25,** un module **2** comprenant un toron **26.**

De manière générale, si N fibres optiques **25** sont à répartir à l'intérieur de la chambre **11** de réaction du photo-bioréacteur **1,** le nombre de modules **2** peut varier de 1 à N avec répartition des N fibres en toron(s).

Ainsi, il est facile de moduler et d'adapter la taille du concentrateur **21** au besoin du photo-bioréacteur 1 et/ou aux micro-organismes.

Dans le cas où des grilles **2g** sont prévues, il n'y a pas un set de grilles par module, mais un set de grilles pour le panneau **3** dans son ensemble, soit au moins trois grilles **2g** par panneau **3.**

### Concentrateur Cassegrain

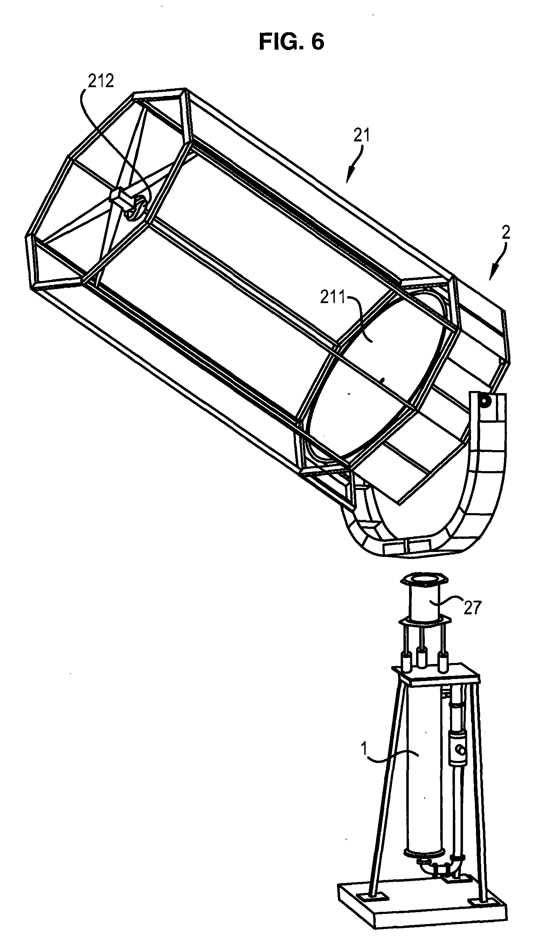

Dans un deuxième mode de réalisation particulière illustré sur la figure 6, le concentrateur **21** peut être constitué d'un système à double paraboles, dit « Cassegrain » comprenant une première parabole **211** et une deuxième parabole **212** plus petite positionnée au foyer de la première parabole **211.**

La première parabole **211** reçoit la lumière du soleil et la renvoie vers la deuxième parabole **212** plus petite, réalisant ainsi une première concentration. La deuxième parabole **212** renvoie ensuite la lumière vers le l'homogénéisateur **22.**

La première parabole **211** peut être orientable lorsqu'elle est associée à un système commandé de suivi du soleil (tracking) bien connu de l'homme du métier. Ceci permet un fonctionnement optimal à chaque moment de la journée.

La deuxième parabole **212** peut également être orientable automatiquement de façon à focaliser ou défocaliser le faisceau concentré **5** pour un contrôle de la densité de flux envoyée vers l'homogénéisateur **22.**

Le filtre **27** est disposé en réception du faisceau concentré 5 renvoyé par la deuxième parabole **212.**

Le filtre **27** peut être un filtre liquide à double parois refroidit. Entre les parois, une solution aqueuse est adjointe et permet à la fois de filtrer le rayonnement ultraviolet indésirable et le rayonnement proche infrarouge.

Le filtre **27** peut encore être un filtre solide (par exemple un filtre plan en verre ou autre filtre décrit ci-dessus pour le concentrateur à lentille de Fresnel, seule la taille et la forme diffèrent) éventuellement refroidi par de l'eau circulant dans une double paroi.

### Photo-bioréacteur

Un exemple de photo-bioréacteur 1 conforme à l'invention comprend un module **2** tel que décrit ci-dessus à modalité avec lentille de Fresnel ou Cassegrain (dans ce cas, un seul module **2** est utilisé) ou au moins deux modules **2** tel que décrit ci-dessus à modalité avec lentille de Fresnel (voir figures 5, 6 et 10)

Le diffuseur **24** est placé à l'intérieur de la chambre **11** de réaction du photo-bioréacteur **1.**

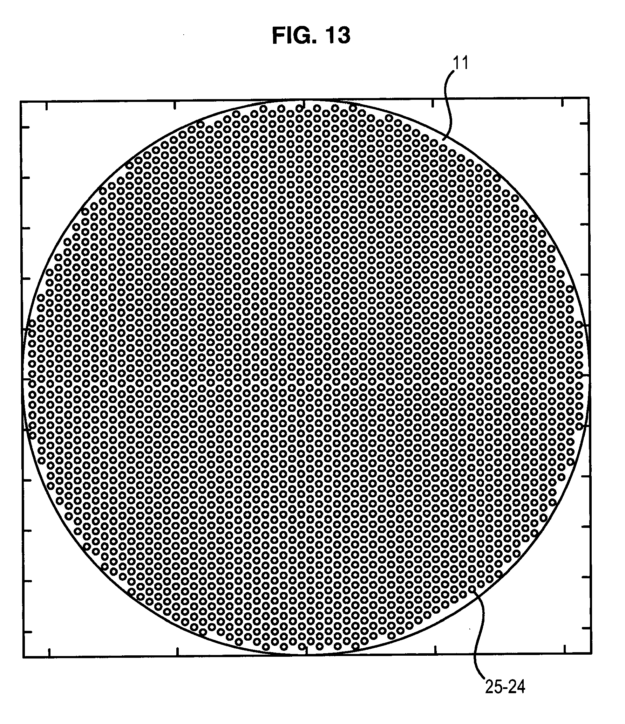

Dans le cas particulier où le guide de lumière **23** et le diffuseur **24** sont formés par un même élément composé de fibres optiques **25** telles que décrites ci-dessus, les parties diffusantes **25-24** des fibres **25** à l'intérieur de la chambre **11** de réaction peuvent être tendues parallèlement entre elles et disposées suivant une maille triangulaire (voir figure 13).

La distance séparant les fibres **25** optiques deux à deux à l'intérieur de la chambre **11** de réaction dépend de la géométrie du photo-bioréacteur **1,** de la concentration en biomasse espérée (intensification) et de la densité de flux recherchée à l'intérieur. Par exemple, pour une densité de flux typique de 4-5 W/m² ( dans le domaine allant de 400 nm à 700 nm), et une concentration en biomasse visée de 15 kg/m³, la distance optimale entre les fibres optiques **25** est de 2,3 mm, l'optimisation de l'ensemble conduisant alors à un diamètre optimal des fibres optiques **25** de 2,3 mm également, quel que soit le diamètre de la chambre **11** de réaction. L'occupation volumétrique optimale totale des fibres optiques **25** à l'intérieur de la chambre 11 de réaction est avantageusement de 23 % environ du volume de la chambre **11** de réaction.

Comme le flux lumineux du faisceau concentré **5** est réparti de manière homogène par l'homogénéisateur **22** dans chacune des fibres optiques **25,** la densité de flux de lumière diffusé par les parties diffusantes **25-24** des fibres optiques **25** est homogène. De plus, comme la surface développée par les fibres optiques à diffusion latérale est très supérieure à la surface de captation, la densité de flux à leur surface est faible. Ceci permet de fonctionner à l'optimum thermodynamique de la photosynthèse, donc d'augmenter les productivités en surface, mais également de diminuer la production de polysaccharides par les micro-organismes. Ainsi, l'adhésion de ces micro-organismes, qui est en partie due à la production des polysaccharides, est minimisée.

Toujours dans ce cas particulier, des grilles **2g** peuvent être prévues pour positionner et maintenir les parties diffusantes des fibres optiques **25** parallèles et suivant un maillage triangulaire à l'intérieur de la chambre **11** de réaction.

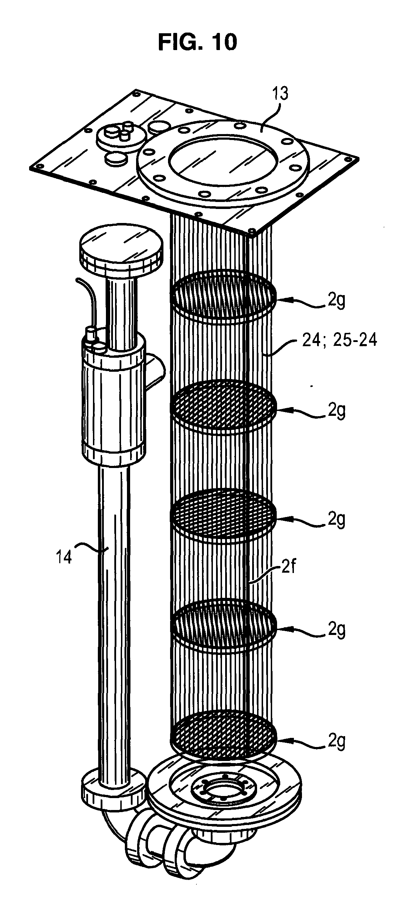

Ainsi, pour obtenir un maillage triangulaire, au moins trois gilles **2g** telles que décrites plus haut sont disposées parallèlement les unes au-dessus des autres à l'intérieur de la chambre **11** de réaction. Deux grilles **2g** successives sont pivotées pour que leurs fils **2g2** respectifs soient tendus suivant des directions différentes et formant un angle de ±60° tel que montré sur la figure 10. Si on compte les grilles **2g** à partir du bas et si l'on prend comme référence la direction des fils de la première grille **2g,** c'est-à-dire que cette direction marque l'angle 0°, alors la direction des fils **2g2** de la deuxième grille **2g** marque l'angle +60°, respectivement -60°. De même, la direction des fils **2g2** de la troisième grille **2g** située au-dessus marque l'angle +120°, respectivement -120° . Si une quatrième grille **2g** est utilisée, la direction de ses fils **2g2** marque l'angle +180°, respectivement -180°, ce qui est équivalent à un angle de 0° .

Les grilles **2g** peuvent être maintenues à distance les unes des autres par des câbles **2f.**

Ainsi, les grilles **2g** forment virtuellement un maillage tubulaire triangulaire. Dans chacune des mailles tubulaires la partie diffusante **25-24** d'une fibre optique **25** peut être placée. La partie diffusante **25-24** de chacune des fibres optiques **25** ainsi placées est maintenue en place assurant ainsi une diffusion du flux lumineux homogène à l'intérieur de la chambre **11** de réaction.

Lorsque des gaines individuelles pour les parties diffusante **25-24** des fibres optiques **25** sont prévues, celles-ci sont moulées au niveau du couvercle **13** par la résine alors qu'elles sont disposées suivant la maille triangulaire telle que décrite plus haut et s'étendent à l'intérieur de la chambre **11** de réaction suivant cette maille triangulaire. Ceci est notamment utilisé dans le cas d'un panneau **3** tel que décrit ci-dessus, dans lequel les torons **26** provenant de plusieurs modules **2** sont réunis au niveau du couvercle **13.**

Les gaines individuelles servent donc de soutien aux fibres optiques **25** à l'intérieur de la chambre **11** de réaction pour maintenir les fibres optiques **25** disposées suivant la maille triangulaire. Dans ce cas, les gaines individuelles à l'intérieur desquelles se trouvent les parties diffusantes **25-24** des fibres optiques **25** passent à travers les gilles **2g,** quand celles-ci sont prévues, de la même manière que les fibres optiques **25** décrites ci-dessus.

La chambre **11** de réaction du photo-bioréacteur **1** peut être à double enveloppe permettant la thermostatisation de la culture de microorganismes. Par exemple, les parois de la chambre **11** de réaction cylindrique sont en inox à double enveloppe de rapport hauteur sur diamètre environ égal à six.

Si un filtre **27** proche infrarouge est utilisé, un excédent de calorie à l'intérieur de la chambre de réaction est évité et il n'est plus nécessaire de prévoir un moyen d'évacuation de calorie pour le photo-bioréacteur **1**.

Un système gazosiphon **14** original à boucle de recirculation externe et à retour central, doublé d'un poreux métallique pour l'injection verticale de gaz (air, CO₂, O₂) est prévu pour le photo-bioréacteur **1** afin d'assurer le mélange de la phase liquide de la culture et les transferts de masse gaz-liquide (CO₂/O₂ principalement) en permettant une bonne circulation des fluides et un bon transfert de masse gaz-liquide dans la chambre **11** de réaction avec un minimum de perte de charges malgré la présence de nombreuses fibres optiques **25.**

Le poreux métallique est placé à la base du système gazosiphon **14** au sein d'une boîte à air qui entoure celui-ci. Ainsi, les gaz sont amenés par une alimentation radiale et injectés verticalement à travers le poreux métallique. Le retour de la solution (liquide) est effectué par le centre du gazosiphon. Le poreux métallique permet un meilleur transfert de masse gaz-liquide en permettant la formation de bulles de gaz plus fines dans la solution.

La circulation efficace engendrée permet alors de minimiser l'adhésion des cellules aux parois et/ou au diffuseur.

## Revendications

1. Module (2) de guidage de la lumière du soleil pour un photo-réacteur (1) comprenant une chambre (11) de réaction, le module (2) comportant :
- un concentrateur (21) disposé en dehors de la chambre (11) à réaction pour concentrer la lumière solaire en un faisceau concentré ;
- un diffuseur (24) à disposer dans la chambre (11) de réaction pour introduire le faisceau concentré à l'intérieur de la chambre (11) de réaction et diffuser le faisceau concentré à l'intérieur de la chambre (11) de réaction ;
**caractérisé en ce que** le module (2) comporte en outre un homogénéisateur (22) disposé entre le concentrateur (21) et le diffuseur (24), en dehors de la chambre (11) de réaction, pour homogénéiser le faisceau concentré avant que le faisceau concentré entre dans le diffuseur (24).

2. Module (2) selon la revendication 1, comprenant en outre un guide de lumière (23) pour guider la lumière du concentrateur (21) au diffuseur (24), le guide de lumière (23) étant disposé entre le l'homogénéisateur (22) et le diffuseur (24) en dehors de la chambre (11) de réaction.

3. Module (2) selon l'une des revendications 1 ou 2, comprenant en outre un filtre (27) proche infrarouge pour filtrer la lumière du soleil de sorte que la lumière transmise dans la chambre 11 de réaction est exempte de rayonnement dans les proches infrarouges.

4. Module (2) selon l'une des revendications 1 à 3, dans lequel le concentrateur (21) est un concentrateur à lentille de Fresnel comprenant une lentille de Fresnel ayant un foyer.

5. Module (2) selon la revendication 4, comprenant en outre un capteur (29) à miroir composé à foyer quasi-ponctuel disposé entre le concentrateur à lentille de Fresnel et l'homogénéisateur (22) et devant le foyer de la lentille de Fresnel pour rediriger le faisceau concentré.

6. Module (2) selon l'une des revendications 1 à 3, dans lequel le concentrateur (21) est un concentrateur de type Cassegrain.

7. Module (2) selon l'une des revendications 1 à 6, dans lequel le guide de lumière (23) et le diffuseur (24) forment un seul et même élément composé de fibres optiques (25) ;
dans lequel chaque fibre optique (25) possède une partie diffusante (25-24), les parties diffusantes (25-24) des fibres optiques (25) formant le diffuseur (24) ; et
dans lequel chaque fibre optique (25) possède une partie de guidage (25-23) en dehors de la chambre (11) de réaction, les parties de guidage (25-23) des fibres optiques (25) formant le guide de lumière (23), les parties de guidage (25-23) des fibres optiques (25) étant rassemblées en un toron (26).

8. Panneau (3) de guidage de lumière comprenant au moins deux modules (2) de guidage de lumière selon l'une des revendications 1 à 5 ou selon la revendication 7 en combinaison avec l'une des revendications 4 ou 5, les modules (2) étant montés en parallèle.

9. Photo-réacteur (1) comprenant une chambre (11) de réaction, **caractérisé en ce qu'**il comprend en outre un module (2) selon l'une des revendications 1 à 7 ou un panneau (3) de guidage de lumière selon la revendication 8, le diffuseur du module (2) ou les diffuseurs du panneau étant disposé(s) à l'intérieur de la chambre (11) de réaction.

10. Photo-réacteur comprenant une chambre (11) de réaction, **caractérisé en ce qu'**il comprend en outre un module (2) selon la revendication 7 ou un panneau (3) de guidage de lumière comprenant un module (2) selon la revendication 7 en combinaison avec l'une des revendications 4 ou 5, les parties diffusantes des fibres optiques (25) du module (2) ou du panneau (3) étant disposées tendues et parallèles entre elles à l'intérieur de la chambre (11) de réaction suivant un maillage triangulaire.

11. Photo-réacteur selon la revendication 10, comprenant en outre au moins trois grilles (2g) disposées parallèlement l'une au-dessus de l'autre, chacune étant formée d'un cadre (2g1) et de fils (2g2) tendus à l'intérieur du cadre (2g1) parallèlement les uns aux autres suivant une direction, la direction des fils d'une grille formant avec la direction des fils d'une grille au-dessus un angle de ±60° afin de maintenir les parties diffusantes (25-24) des fibres optiques (25) selon un maillage triangulaire.

12. Photo-réacteur selon l'une des revendications 10 ou 11, comprenant en outre un gazosiphon (14) à boucle de recirculation externe et un retour central pour mélanger efficacement le contenu de la chambre (11) de réaction.

## Patentansprüche

1. Modul (2) zum Leiten von Sonnenlicht für einen Photoreaktor (1), der eine Reaktionskammer (11) aufweist, wobei das Modul (2) aufweist:
- einen außerhalb der Reaktionskammer (11) angeordneten Konzentrator (21) zum Konzentrieren des Sonnenlichts in ein konzentriertes Bündel;
- einen in der Reaktionskammer (11) anzuordnenden Diffusor (24) zum Einspeisen des konzentrierten Bündels in das Innere der Reaktionskammer (11) und zum Streuen des konzentrierten Bündels im Innern der Reaktionskammer (11);
**dadurch gekennzeichnet, dass** das Modul (2) ferner einen zwischen dem Konzentrator (21) und dem Diffusor (24) außerhalb der Reaktionskammer (11) angeordneten Homogenisator (22) aufweist, um das konzentrierte Bündel zu homogenisieren, bevor das konzentrierte Bündel in den Diffusor (24) eintritt.

2. Modul (2) nach Anspruch 1, ferner mit einem Lichtleiter (23) zum Leiten des Lichts vom Konzentrator (21) zum Diffusor (23), wobei der Lichtleiter (23) zwischen dem Homogenisator (22) und dem Diffusor (24) außerhalb der Reaktionskammer (11) angeordnet ist.

3. Modul (2) nach einem der Ansprüche 1 oder 2, ferner mit einem Filter (27) für nahes Infrarot, um das Sonnenlicht zu filtern, derart, dass das in die Reaktionskammer (11) durchgelassene Licht frei von Strahlung der nahen Infrarotbereiche ist.

4. Modul (2) nach einem der Ansprüche 1 bis 3, wobei der Konzentrator (21) ein Fresnellinse-Konzentrator ist, der eine Fresnellinse mit einem Brennpunkt aufweist.

5. Modul (2) nach Anspruch 4, ferner mit einem Kollektor (29) der Bauart eines zusammengesetzten Spiegels mit quasi punktförmigem Brennpunkt, der zwischen dem Fresnellinse-Konzentrator und dem Homogenisator (22) und vor dem Brennpunkt der Fresnellinse angeordnet ist, um das konzentrierte Bündel umzulenken.

6. Modul (2) nach einem der Ansprüche 1 bis 3, wobei der Konzentrator (21) ein Konzentrator des Cassegrain-Typs ist.

7. Modul (2) nach einem der Ansprüche 1 bis 6, wobei der Lichtleiter (23) und der Diffusor (24) ein aus Faseroptiken (25) zusammengesetztes einziges Element bilden;
wobei jede Faseroptik (25) einen streuenden Teil (25-24) aufweist und die streuenden Teile (25-24) der Faseroptiken (25) den Diffusor (24) bilden; und
wobei jede Faseroptik (25) einen Leitungsteil (25-23) außerhalb der Reaktionskammer (11) aufweist, wobei die Leitungsteile (25-23) der Faseroptiken (25) den Lichtleiter (23) bilden und die Leitungsteile (25-23) der Faseroptiken (25) zu einer Litze (26) zusammengefasst sind.

8. Lichtleitungspanel (3) mit mindestens zwei Lichtleitungsmodulen (2) nach einem der Ansprüche 1 bis 5 oder nach Anspruch 7 in Kombination mit einem der Ansprüche 4 oder 5, wobei die Module (2) parallel angeordnet sind.

9. Photoreaktor (1) mit einer Reaktionskammer (11), **dadurch gekennzeichnet, dass** er ferner ein Modul (2) nach einem der Ansprüche 1 bis 7 oder ein Lichtleitungspanel (3) nach Anspruch 8 aufweist, wobei der Diffusor des Moduls (2) oder die Diffusoren des Panels im Innern der Reaktionskammer (11) angeordnet ist oder sind.

10. Photoeaktor mit einer Reaktionskammer (11), **dadurch gekennzeichnet, dass** er ferner ein Modul (2) nach Anspruch 7 oder ein Lichtleitungspanel (3) mit einem Modul (2) nach Anspruch 7 in Kombination mit einem der Ansprüche 4 oder 5 aufweist, wobei die streuenden Teile der Faseroptiken (25) des Moduls (2) oder des Panels (3) straff gespannt und parallel zueinander im Innern der Reaktionskammer (11) gemäß einem dreieckigen Netzwerk angeordnet sind.

11. Photoreaktor nach Anspruch 10, ferner mit mindestens drei parallel übereinander angeordneten Gittern (2g), von denen jedes aus einem Rahmen (2g1) und Drähten (2g2) besteht, die innerhalb des Rahmens (2g1) parallel zueinander in eine Richtung straff gespannt sind, wobei die Richtung der Drähte eines Gitters mit der Richtung der Drähte eines darüber liegenden Gitters einen Winkel von ±60° bildet, um die streuenden Teile (25-24) der Faseroptiken (25) gemäß einem dreieckigen Netzwerk zu halten.

12. Photoreaktor nach einem der Ansprüche 10 oder 11, ferner mit einem Gassiphon (14) mit externer Umwälzungsschleife und einem zentralen Rücklauf, um wirkungsvoll den Inhalt der Reaktionskammer (11) zu mischen.

## Claims

1. A module (2) for guiding sunlight for a photoreactor (1) comprising a reaction chamber (11), the module (2) comprising:
- a concentrator (21) placed outside the reaction chamber (11) in order to concentrate the sunlight in a concentrated beam;
- a scattering device (24) to be placed inside the reaction chamber (11) in order to introduce the concentrated beam into the reaction chamber (11) and diffuse the concentrated beam in the reaction chamber (11) ;
**characterised in that** the module (2) also comprises a homogeniser (22) placed between the concentrator (21) and the scattering device (24), outside the reaction chamber (11), in order to make the concentrated beam homogeneous before the concentrated beam enters the scattering device (24).

2. The module (2) of claim 1, also comprising a light guide (23) for guiding the light from the concentrator (21) to the scattering device (24), the light guide (23) being placed between the homogeniser (22) and the scattering device (24) outside the reaction chamber (11).

3. The module (2) of claim 1 or claim 2, also comprising a near-infrared filter (27) for filtering sunlight so that the light transmitted into the reaction chamber 11 is free from radiation in the near infrared.

4. The module (2) of one of claims 1 to 3, wherein the concentrator (21) is a Fresnel-lens concentrator comprising a Fresnel lens having a focus.

5. The module (2) of claim 4, also comprising a compound-mirror collector (29) with an almost punctiform focus placed between the Fresnel-lens concentrator and the homogeniser (22) and in front of the focus of the Fresnel lens in order to redirect the concentrated beam.

6. The module (2) of one of claims 1 to 3, wherein the concentrator (21) is a concentrator of the Cassegrain type.

7. The module (2) of one of claims 1 to 6, wherein the light guide (23) and the scattering device (24) form a single element composed of optical fibres (25);
wherein each optical fibre (25) has a diffusing part (25-24), the diffusing parts (25-24) of the optical fibres (25) forming the scattering device (24); and
wherein each optical fibre (25) has a guide part (25-23) outside the reaction chamber (11), the guide parts (25-23) of the optical fibres (25) forming the light guide (23), the guide parts (25-23) of the optical fibres (25) being collected together into a strand (26).

8. A light-guide panel (3) comprising at least two modules (2) of one of claims 1 to 5 or claim 7 in combination with one of claims 4 or 5, the modules (2) being mounted in parallel.

9. A photoreactor (1) comprising a reaction chamber (11), **characterised in that** it also comprises a module (2) of one of claims 1 to 7 or a light-guide panel (3) according to claim 8, the scattering device of the module (2) or the diffusers of the panel being placed inside the reaction chamber (11).

10. A photoreactor comprising a reaction chamber (11), **characterised in that** it also comprises a module (2) according to claim 7 or a light-guide panel (3) comprising a module (2) according to claim 7 in combination with one of claims 4 or 5, the diffusing parts of the optical fibres (25) of the module (2) or of the panel (3) being arranged tensioned and parallel to each other in the reaction chamber (11) into a triangular mesh.

11. The photoreactor of claim 10, also comprising at least three gratings (2g) placed parallel one above another, each being formed by a frame (2g1) and tensioned wires (2g2) in the frame (2g1) parallel to one another in one direction, the direction of the wires of a grating forming with the direction of the wires of another grating above an angle of ±60° to hold the diffusing parts (25-24) of the optical fibres (25) in a triangular mesh.

12. The photoreactor of one of claims 10 or 11, also comprising a gas trap (14) with an external recirculation loop and a central return for mixing the content of the reaction chamber (11) effectively.
